# EUROPEAN PATENT APPLICATION

(11) **EP 1 652 523 A1**
(43) Date of publication of application: **03.05.2006**
(21) Application number: 04748201.3
(22) Date of filing: 03.08.2004
(51) Int. Cl.: A61K 31/4174, A61K 9/70, A61K 47/06, A61K 47/10, A61K 47/12, A61K 47/14, A61K 47/30, A61K 47/44, A61P 13/10, C07D 233/61

(54) **TRANSDERMAL ABSORPTION PREPARATION**

(30) Priority: 04.08.2003 JP 2003286103
(71) Applicant: KYORIN PHARMACEUTICAL CO., LTD., Tokyo 101-8311 (JP); ONO PHARMACEUTICAL CO., LTD., Osaka-shi, Osaka 541-8526 (JP)
(72) Inventor: NAKASHIMA, Katashi, Tatebayashi-shi, Gunma 3740016 (JP); OHMORI, Satoshi, Tochigi-shi, Tochigi 3280051 (JP); KANAYAMA, Norihiro, Shimotsuga-gun, Tochigi 329-0101 (JP); SAKAI, Yoshiki, c/o Ono Pharmaceutical Co., Ltd., Mishima-gun, Osaka 618-8585 (JP)
(74) Representative: Vossius & Partner
(86) International application number: PCT/JP2004/011068
(87) International publication number: WO 2005/011683

(57) **Abstract**

(Object) A transdermal preparation is provided, which ensures stable and effective absorption of 4-(2-methyl-1-imidazolyl)-2,2-diphenylbutylamide (KRP-197) which has a low skin absorption and is a bladder-selective muscarinic M3 and M1 receptor antagonist, into body through the skin while causing little side effects and providing sustained pharmacological effect with less skin irritancy. (Solving means) A composition comprising KRP-197 and an external preparation base is deposited and dried onto a structural body or a small pool of the composition is deposited on the structural body to obtain a single adhesive layer-type transdermal preparation or a reservoir-type transdermal preparation. These preparations can ensure high permeation of KRP-197 through the skin and sustained absorption of KRP-197 into body while causing decreased skin irritancy.

## Description

### TECHNICAL FIELD

The present invention relates to a transdermal preparation containing as an active ingredient 4-(2-methyl-1-imidazolyl)-2,2-diphenylbutylamide (which is referred to as KRP-197, hereinafter), a bladder-selective muscarinic M3 and M1 receptor antagonist used as a treatment for increased urinary frequency and urinaryincontinence that occur in patients with overactive bladder.

### BACKGROUND ART

KRP-197 is a novel selective muscarinic M3 and M1 receptor antagonist (Patent Document 1) and is considered a potential cure for increased urinary frequency and urinary incontinence (Non-Patent Document 1).

Like other commercially available drugs for treating increased urinary frequency and urinary incontinence, KRP-197 is provided in the form of oral solid preparation (i.e., tablets) (Patent Document 2).
[Patent Document 1] Japanese Patent Laid-Open Publication No. Hei 7-15943
[Patent Document 2] WO01/34147 A1 Pamphlet
[Non-Patent Document 1] Bioorg. Med. Chem., 1999, 7, 1151-1161.

### DISCLOSURE OF THE INVENTION

### PROBLEMS TO BE SOLVED BY THE INVENTION

Recent advances in medicine have led to the emergence of an aging population, affecting many aspects of modern society. For example, approximately half of the aged people who are bedridden or suffering from dementia and are in need of care are said to experience increased urinary frequency and urinary incontinence. It is estimated that more people will suffer from increased urinary frequency and urinary incontinence and the type of the patients with these symptoms will be shifted in the future. Increased urinary frequency and urinary incontinence often occur to long-term bedridden patients who have experienced cerebral vascular accidents, such as cerebral infarction and cerebral hemorrhage, brain and spinal damage or various tumor operations. Elderly people often have difficulty in swallowing, which makes it difficult to administer oral preparations designed to be taken at regular intervals to these patients. Since oral administration exposes the liver to high concentrations of drugs and increases the highest blood concentration of drug, the chance of side effects is increased. Thus, more patient-oriented non-oral preparations are desired so as to improve the quality of lives of the patients to whom oral administration is hardly applied.

Among possible forms of non-oral preparations are injections and external preparations (such as liquids, ointments, patches, and infusions). While having immediate pharmacological effects, injections require careful control of delivery methods and doses by medical personnel as compared to other preparations and are associated with burdensome problems such as difficulty in administering at home, accompanying pain and inability to eliminate drug that has once been administered. On the other hand, external preparations are less burdensome to patients as compared to the injections since the external preparations can be applied at home, accompany no pain and can eliminate drug after application. Nonetheless, some drawbacks still remain: External preparations are generally applied by patients themselves or their caretakers , so that the poorly controlled drug delivery or dosage may lead to a reduced chance of proper drug use. In cases of external patches, skin rash may result. Thus, injections and conventional external preparations are not sufficiently effective for use with patients of a wide age range and their caretakers .

Recently, transdermal preparations that employ the concept of drug delivery system to achieve optimum treatment have attracted much attention and have been intensively studied. Such preparations have overcome the above-described drawbacks of conventional external preparations and, at the same time, offer sustained drug efficencies and reduced side effects, distinctive features that have never been achieved by conventional external preparations, along with the advantages of the conventional external preparations. Of different routes of administration, the transdermal drug delivery system, including ointments and patches, has attracted significant attention. Drugs delivered through this system directly enter subdermal capillary veins and heart and do not exposed to the first-pass effect in liver, so that they are transported through arteries and veins to the target site without being metabolized or decomposed. Thus, this system ensures high bioavailability of drugs. The system requires relatively small amounts of drugs and can serve as an effective sustained drug-release technique. This route of administration also has the following advantages: The side effects caused by temporarily high blood concentrations of the drug occurring in administration of oral preparations and injections can be avoided; transdermal preparations can ensure sustained drug-absorption from the skin, thus requiring a fewer number of times of administration than the oral preparations; and the application of transdermal preparations can be terminated immediately in the event of side effect.

Nevertheless, the barrier function of the skin reduces the drug absorption from the skin and often prevents delivery of effective doses of drugs by a patch with a practical surface area. In addition, drugs, which are often hydrophilic compounds or salts, have relatively high polarity and, thus, can hardly be absorbed from the skin. The most effective barrier against the transdermal drug absorption is the stratum corneum, a complex structure of keratinized small cell residues separated by the extracellular lipid area. The stratum corneum has significantly lower permeability to drugs than the mucosa in the mouth or stomach.

Many attempts have been made to circumvent the foregoing drawbacks of transdermal delivery system: One technique involves increasing the drug concentration in the transdermal delivery system to increase the proportion of the drug distributed to the skin. In another technique, a material that is less permeable to water is used to block the release of perspiration and moisture, thereby hydrating the skin. Still another technique uses a transdermal absorption enhancer that reduces the barrier function of the skin against materials outside. Other techniques are also known involving sonication and electric currents. Among transdermal preparations employing the transdermal delivery system and developed and marketed to date are those intended to deliver nitroglycerin, isosorbid dinitrate, estradiol, scopolamine, clonidine, nicotine, testosterone, fentanyl, tulobuterol, and other drugs.

Similarly, transdermal preparations are expected to be applied to the delivery of therapeutic agents for increased urinary frequency and urinary incontinence. Specifically, there is a need for a preparation that can maintain high blood concentrations of muscarinic receptor antagonists for a prolonged period of time. Although several external transdermal preparations have been proposed recently, problems associated with the stability, constant blood concentration, pharmacological effect, adherence, discomfort, and skin irritancy of the preparation still remain.

Muscarinic receptor antagonists used in the treatment of increased urinary frequency and urinary incontinence are generally known to cause mouth dryness, constipation, stomach ache, difficulty in urination, urinary retention, dilatation of pupils, and other side effects, posing a serious clinical problem. Thus, a search was conducted for bladder-selective muscarinic M3 and M1 receptor antagonists and led to the identification of KRP-197. Since then, the possibility of its use as a treatment for increased urinary frequency and urinary incontinence that occur in patients with overactive bladder has been discussed. However, even KRP-197 may cause side effects when it is orally administered and the blood concentration of drug is temporarily increased. Transdermal delivery system, which ensured stable and sustained blood concentrations, seemed to offer an answer to this problem. However, transdermal absorption of KRP-197 is significantly low when the drug is used alone in a transdermal preparation, and it has been considered difficult to achieve effective blood concentrations of the drug.

The present invention addresses the above-described problem, and it is thus an object of the present invention to provide an effective transdermal preparation that ensures stable and sustained absorption of muscarinic receptor antagonists, in particular KRP-197, into body while causing little skin irritancy and providing sufficient pharmacological effect.

### MEANS FOR SOLVING THE PROBLEMS

In an effort to achieve the aforementioned object, the present inventors have discovered that a transdermal preparation containing a muscarinic receptor antagonist as an active ingredient and comprising an external preparation base and a structural body can achieve this objective. It is this discovery that led to the present invention.

Specifically, the present inventors have discovered that a single adhesive layer-type transdermal preparation or a reservoir-type transdermal preparation obtained by depositing a mixture of KRP-197 and an external preparation base onto a structural body can ensure high permeation of KRP-197 through the skin and sustained absorption of KRP-197 into body while causing decreased skin irritancy. The mixture is deposited onto the structural body by spreading it over the structural body and drying it or by depositing a small pool of the mixture on the structural body.

Accordingly, the present invention concerns the following:
(1) A transdermal preparation containing a muscarinic receptor antagonist and an external preparation base;
(2) The preparation according (1) above, wherein the muscarinic receptor antagonist is one or two or more selected from 4-(2-methyl-1-imidazolyl)-2,2-diphenylbutylamide, (+)-(R)-2-[α-[2-(diisopropylamino)ethyl]benzoyl]-p-cresol tartrate, (S)-1-[2-(2,3-dihydro-5-benzofuranyl)ethyl]-α,α-diphenyl-3-pyrrolidine acetamide hydrobromide, (+)-(1S,3'R)-quinuclidin-3'-yl-phenyl-1,2,3,4-tetrahydroisoquinoline-2-carboxylate succinate, 4-diethylamino-2-butynyl(±)-α-cyclohexyl-α-phenylglycolate hydrochloride, 1-methyl-4-piperidyl-α,α-diphenyl-α-n-propoxyacetate hydrochloride, (±)-N-t-butyl-1-methyl-3,3-diphenylpropylamine hydrochloride, and 2-piperidinoethyl-3-methyl-4-oxo-2-phenyl-4H-1-benzopyran-8-carboxylate;
(3) The preparation according to (2) above, wherein the muscarinic receptor antagonist is 4-(2-methyl-1-imidazolyl)-2,2-diphenylbutylamide;
(4) The preparation according to (1) above for use as a prophylactic or therapeutic agent for preventing or treating a disorder in patients with overactive bladder, the disorder selected from increased urinary frequency, urinary incontinence, asthma, chronic airway obstruction, and irritable bowel syndrome;
(5) The preparation according to (4) above, wherein the disorder is increased urinary frequency and urinary incontinence in patients with overactive bladder;
(6) The preparation according to (1) above, provided in the form of patch;
(7) The preparation according to (6) above, provided in the form of a single adhesive layer-type or a reservoir-type transdermal preparation;
(8) The preparation according to (7) above, containing as an active ingredient a dissolved form of 4-(2-methyl-1-imidazolyl)-2,2-diphenylbutylamide alone;
(9) The preparation according to (7) above, containing as active ingredients dissolved and non-dissolved forms of 4-(2-methyl-1-imidazolyl)-2,2-diphenylbutylamide;
(10) The preparation according to (1) above, wherein the external preparation base is a base or combination of bases selected from the group consisting of an amphipathic solubilizing agent, a suspension base, a softener, an emulsifier, a buffer, a transdermal permeability enhancer, a tackifier, a tackiness enhancer, an adhesive, a skin irritancy mitigator, and an additive;
(11) The transdermal preparation for treating increased urinary frequency and urinary incontinence according to (1) above, containing as an active ingredient 4-(2-methyl-1-imidazolyl)-2,2-diphenylbutylamide and comprising an external preparation base and a structural body;
(12) The transdermal preparation for treating increased urinary frequency and urinary incontinence according to (11) above, which is of a single adhesive layer type, comprising:
   an adhesive layer formed of 4-(2-methyl-1-imidazolyl)-2,2-diphenylbutylamide in conjunction with a single or combination of the external preparation bases; and
   a structural body comprising a support and a peelable liner;
(13) The transdermal preparation for treating increased urinary frequency and urinary incontinence according to (11) above, which is of a reservoir type, comprising:
   a mixture of 4-(2-methyl-1-imidazolyl)-2,2-diphenylbutylamide and a single or combination of the external preparation bases; and
   a structural body comprising a membrane for controlling drug permeation, an adhesive layer, a support, and a peelable liner; and
(14) The transdermal preparation according to any one of (11) to (13) above, wherein the external preparation base comprises a compound or combination of compounds selected from the group consisting of a water-soluble polymer, a fat-soluble polymer, a fatty acid, a fatty acid ester, a fatty acid metal salt, animal or plant fats and oils, an alcohol, a terpene compound, and water.

### ADVANTAGE OF THE INVENTION

Although muscarinic receptor antagonists are hardly absorbed through the skin, the transdermal preparation provided in accordance with the present invention ensures stable, effective and sustained absorption of muscarinic receptor antagonists, in particular KRP-197, into body while being less irritant to the skin.

### BRIEF DESCRIPTION OF THE DRAWINGS

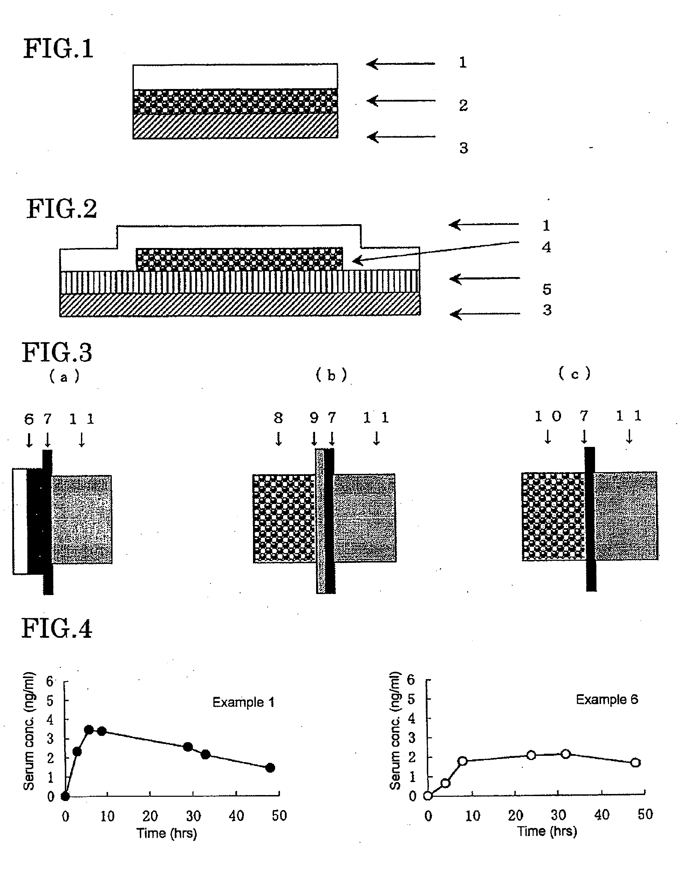

One example of cross-sectional view of a single adhesive layer-type transdermal preparation is shown in Fig. 1 .
One example of cross-sectional view of a reservoir-type transdermal preparation is shown in Fig. 2..
A diagram of KRP-197 preparations used in an *in vitro* skin permeability test is shown in Fig. 3, where (a) shows a single adhesive layer-type transdermal preparation used in Examples 1 through 4, (b) shows a preparation used in Example 5, and (c) shows a preparation used in Comparative Example 1.
Fig. 4 shows the serum concentration profiles of KRP-197 in male hairless rats applied preparations of Examples 1 and 6.

### INDICATION OF REFERENCE NUMERALS

1. Bucking
2. adhesive layer
3. peelable liner
4. Drug reservoir
5. adhesive layer (also serves as a membrane to control the permeability of drug)
6. single adhesive layer-type transdermal preparation
7. shaved abdominal skin of male hairless rat
8. KRP-197 dispersion in a cell on the stratum corneum side
9. drug permeability control membrane
10. sample solution for skin permeability test placed in a cell on the stratum corneum side. The solution contains KRP-197 alone.
11. PBS (pH 7.4) containing 1% gentamicin sulfate placed in a cell on the dermic layer side.

### BEST MODE FOR CARRYING OUT THE INVENTION

4-(2-methyl-1-imidazolyl)-2,2-diphenylbutylamide, or KRP-197, the active ingredient of transdermal preparation of the present invention, is a bladder-selective muscarinic M3 and M1 receptor antagonist and can be used as a treatment for increased urinary frequency and urinary incontinence.

The active ingredient of the transdermal preparation of the present invention, KRP-197 may be used either as a free form or as a medically acceptable salt. It is used in any pharmaceutically effective amount for transdermal preparations. While the amount of KRP-197 used may vary depending on the form of transdermal preparation and the type and amount of the external preparation base used in the transdermal preparation, the active ingredient is preferably used in an amount of about 0.01 to about 10wt% relative to the weight of the solid components.

While the external preparation base for use in the transdermal preparation of the present invention may be any base commonly used in external preparations, it is preferably a water-soluble polymer (e.g., polyacrylic acids and derivatives thereof, cellulose derivatives, polyvinyl alcohols, gelatin, casein, polyethylene glycol, gum Arabic, methyl vinyl ether/maleic anhydride copolymers, and naturally-occurring polysaccharides), fat-soluble polymers (e.g., natural rubbers, isoprene rubber, butyl rubber, styrene-isobutylene block copolymers, styrene-butadiene copolymers, silicone, lanoline, vaseline, plastibase, beeswax, cetaceum, and solid paraffin), fatty acids and derivatives thereof (e.g., fatty acids having 3 to 40 carbon atoms, esters and alkaline metal salts thereof), animal and plant fats and oils (e.g., olive oil, mint oil, soybean oil, cottonseed oil, corn oil, eucalyptus oil, castor oil, sesame oil, almond oil, camellia oil, apricot kernel oil, mink oil, safflower seed oil, and coconut oil), alcohols (e.g., alcohols having 1 to 40 carbon atoms and 1 to 10 hydroxyl groups, such as ethanol, glycerol, propylene glycol, polyethylene glycol, octane diol, butane diol, and D-sorbitol), terpene compounds (menthol, menthone, limonene, pinene, piperitone, teripinene, terpinolene, terpinol, and carveol), surfactants (e.g., nonionic surfactants, anionic surfactants, and cationic surfactants) and water. These external preparation bases may be used either individually or in combination.

The external preparation base may be a base or combination of bases selected from the group consisting of an amphipathic solubilizing agent, a suspension base, a softener, an emulsifier, a buffer, a transdermal permeability enhancer, a tackifier, a tackiness enhancer, an adhesive, a skin irritancy mitigator, and an additive. These bases may be used in conjunction with the above-described external preparation bases.

Examples of the amphipathic solubilizing agent include N-methyl-2-pyrrolidone, L-menthol, methyl ethyl ketone, methyl isobutyl ketone, higher fatty acid esters (e.g., isopropyl myristate (IPM), isopropyl palmitate, and oleyl oleate), diethanolamide laurate, triethyl citrate, dimethyl imidazolidinone, glycerol fatty acid esters (e.g., lipophilic glycerol monooleate), polyglycerol fatty acid esters, sorbitan fatty acid esters (e.g., sorbitan monooleate, sorbitan trioleate, and sorbitan sesquioleate), polyoxyethylene sorbitan esters, polyoxyethylene sorbitol fatty acid esters (e.g., polysorbate 20, polysorbate 60, polysorbate 80, and polyoxyethylene sorbitan monolaurate), polyoxyethylene alkylformaldehyde condensates, polyoxyethylene sterols/hydrogenated sterols, polyoxyethyleneglycol fatty acid esters, polyoxyethylene lanolin, sodium phosphate polyoxyethylene lauryl ether, polyoxyethylene alkyl ethers (e.g., lauromacrogol), polyoxyethylenepolyoxypropylene alkyl ethers, polypropylene glycol 2000, polyoxyethylene polyoxypropylene glycol (e.g., polyoxyethylene (160) polyoxypropylene (30) glycol and polyoxyethylene (196) polyoxypropylene (67) glycol), polyoxyethylene alkylphenyl ethers (e.g., polyoxyethylene nonylphenyl ether), polyvinyl alcohol, beeswax derivatives, polyoxyethylene alkylamine·fatty acid amides, polyoxyethylene alkyl ether phosphoric acids· phosphates, monofatty acid polyoxyethylene hydrogenated castor oil, polyoxyethylene castor oil, polyoxyethylene hydrogenated castor oil, polyoxyethylene hydrogenated castor oil 10, polyoxyethylene hydrogenated castor oil 40, polyoxyethylene hydrogenated castor oil 50, polyoxyethylene hydrogenated castor oil 60, polyethyleneglycol monooleate, polyethyleneglycol monostearate, ethoxy glycol, propylene glycol, 1,3-butylene glycol, polyethylene glycol 400, methanol, acetone, ethyl acetate, ethyl lactate, triacetin, pantotenyl ethyl ether, ethyleneglycol monobutyl ether, dimethylether , diethylether , monoethanolamine, diethanolamine, diethylamine, isopropanolamine, diisopropanolamine, triethanolamine, 2-amino-2-methyl-1-propanol, 2-amino-2-methyl-1-propanediol, N,N-dimethylacetamide, dimethyl sulfoxide, dodecyl sulfoxide, geraniol denatured alcohol, 8-acetyl sucrose denatured alcohol, linaryl acetate denatured alcohol, phenylethylalcohol denatured alcohol, benzyl alcohol, butanol, 2-butanol, glycerol, higher alcohols (e.g., lauryl alcohol, isopropanol, isostearyl alcohol, octyldodecanol, and oleyl alcohol), geraniol, diethylene glycol, ethoxydiglycol, diisopropylene glycol, propylene glycol fatty acid ester, propylene glycol monocaprate (S-218), propylene carbonate, thioglycol acid, propionic acid, methanesulfonic acid, glacial acetic acid, lactic acid, butyric acid, citric acid, hydrochloric acid, phosphoric acid, dibasic sodium phosphate, potassium dihydrogen phosphate, potassium iodide, fatty acids (e.g., capric acid, adipic acid, sebacic acid, myristic acid, and oleic acid), ichthammol, benzyl benzoate, nicotinic acid amide, nicotinic acid benzyl ester, dibasic acid diesters (diethyl sebacate, diisopropyl sebacate, and diisopropyl adipate), colza oil, soybean oil, soybean lecithin, D-mannitol, zinc sulfate, aluminum sulfate, sodium thiosulfate, medium chain fatty acid triglyceride, β-cyclodextrin, and liquid paraffin.

Examples of the suspension base include ethanol, stearyl alcohol, cetanol, polyols (e.g., ethylene glycol, propylene glycol, butanediol, triethylene glycol, polyethylene glycol (e.g., macrogol 200, macrogol 300, macrogol 400, macrogol 4000, macrogol 600, and macrogol 1500), cetomacrogol 1000, glycerol, and ethyleneglycol monostearate), higher fatty acid esters (e.g., hexyl laurate, butyl stearate, isopropyl palmitate, isopropyl myristate, octyldodecyl myristate, myristyl myristate, glycerol fatty acid esters, glycerol monostearate, and sorbitan fatty acid esters), sorbitan monolaurate, polyoxyethylene sorbitan monolaurate, sorbitan trioleate, propylene glycol fatty acid esters, polyoxyethylene sorbitol fatty acid esters (*e.g.,* polysorbate 20, polysorbate 60, and polysorbate 80), sorbitan sesquioleate, polyoxyethylene nonylphenyl ether, polyoxyethylene hydrogenated castor oil, polyoxyethylene hydrogenated castor oil 60, polyoxyethylene (160) polyoxypropylene (30) glycol, macrogol 6000, dioctylsodium sulfosuccinate, dimethylpolysiloxane·silicon dioxide mixture, carboxyvinylpolymer, methyl cellulose, hydroxypropyl cellulose, carboxymethylcellulose, carboxymethylcellulose sodium, crystalline cellulose·carmellose sodium, povidone, sodium methylene-β-naphthalene sulfonate, sodium erythorbate, liquid hydrocarbons (*e.g*., liquid paraffin), animal and plant fats and oils (*e.g.*, almond oil, camellia oil, apricot kernel oil, mink oil, safflower seed oil, coconut oil, eucalyptus oil, soybean oil, sesame oil, corn oil, rapeseed oil, sunflower oil, cottonseed oil, olive oil, castor oil, peanut oil, and wheat germ oil), soybean lecithin, beeswax, white beeswax, hydrogenated oil, squalane, squalene, medium chain fatty acid triglyceride, gum Arabic, gum Arabic powder, xanthan gum, pectin, bentonite, sodium alginate, alginic acid propylene glycol ester, sodium chloride, benzalkonium chloride, kaolin, carrageenan carnauba wax, dried aluminum hydroxide gel, aluminum magnesium silicate, magnesium aluminometasilicate, potassium hydroxide, sodium hydroxide, aluminum stearate, and phosphate.

Examples of the softener include allantoin, almond oil, olive oil, rapeseed oil, castor oil, cottonseed oil/soybean oil mixture, processed oil, tallow, propylene glycol, polybutene, glycerol, liquid paraffin, light liquid paraffin, crystalline cellulose, macrogol 1500, squalane, squalene, purified lanolin, medium chain fatty acid triglyceride, glycerol monostearate, isopropyl myristate, crude rubber, cetyl lactate, vanilyl amide nonylate, and high-cis-polyisoprene rubber.

Examples of the emulsifier include glycerol fatty acid esters (e.g., glycerol monooleate, lipophilic glycerol monooleate, glycerol monostearate, lipophilic glycerol monostearate, self-emulsifying glycerol monostearate, polyoxyethylene glyceryl triisostearate, and glycerol monooleate·glycerol dioleate·propylene glycol mixed emulsifier), sorbitan fatty acid esters, sorbitan monooleate, sorbitan trioleate, sorbitan monolaurate, sorbitan sesquioleate, sorbitan monostearate, sorbitan tristearate, sorbitan monopalmitate, glycerol fatty acid esters, propylene glycol fatty acid esters, polyglycerol fatty acid esters, polyoxyethylene glycerol fatty acid esters, polyoxyethylene glycerol monostearate, polyoxyethylene sorbitol fatty acid esters (*e.g*., polysorbate 20, polysorbate 40, polysorbate 60, polysorbate 65, polysorbate 80, polyoxyethylene sorbitan monolaurate, polyethylene sorbitan monooleate, polyoxyethylene sorbitan trioleate, and polyoxyethylene sorbitol tetraoleate), cetomacrogol 1000, macrogol 20000, macrogol 300, macrogol 400, polyoxyethylene alkyl ether, polyoxyethylenecetyl ether, polyoxyethylene nonylphenyl ether, polyoxyethylene octylphenyl ether, polyoxyethylene oleyl ether, polyoxyethylene cetostearyl ether, polyoxyethylene sorbitol beeswax, sodium polyoxyethylene oleyl ether phosphate, sodium polyoxyethylene cetyl ether phosphate, polyoxyethylene lanolin, lauromacrogol, polyoxyethylene glycol fatty acid esters, polyoxyethylene castor oil, polyoxyethylene hydrogenated castor oil, polyoxyethylene hydrogenated castor oil 5, polyoxyethylene hydrogenated castor oil 10, polyoxyethylene hydrogenated castor oil 20, polyoxyethylene hydrogenated castor oil 40, polyoxyethylene hydrogenated castor oil 50, polyoxyethylene hydrogenated castor oil 60, polyoxyethylene behenyl ether, α-monoisostearyl glyceryl ether, polyoxyethylene stearyl ether phosphate, polyoxyethylene (160) polyoxypropylene (30) glycol, polyoxyethylene (1) polyoxypropylene (1) cetyl ether, polyoxyethylene (10) polyoxypropylene (4) cetyl ether, polyoxyethylene (20) polyoxypropylene (4) cetyl ether, polyoxyethylene (20) polyoxypropylene (8) cetyl ether, propylene glycol, polyethylene glycol monooleate, ethylene glycol monostearate, polyethylene glycol monostearate, propylene glycol monostearate, polyethylene glycol distearate, polyethylene glycol monolaurate, diethanolamide laurate, fatty acid esters, isopropyl myristate, octyldodecyl myristate, sucrose fatty acid ester, carboxyvinylpolymer, methylcellulose , sodium carboxymethylcellulose, hydoxypropyl cellulose, methyl acrylate·acrylic acid-2-ethylhexyl copolymer resin emulsion, dioctyl sodium sulfosuccinate, liquid hydrocarbons (e.g., liquid paraffin and paraffin), animal oils, plant oils, mineral oils, cottonseed oil·soybean oil mixture, egg yolk oil, egg yolk phospholipid, hydrocarbons, fatty acids (e.g., stearic acid), potassium stearate, sodium stearate, polyoxyl stearate 40, polyoxyl stearate 45, polyoxyl stearate 55, coconut oil fatty acid diethanolamide, higher alcohol silicone oil, beeswax, white beeswax, paraffin wax, cetaceum, squalane, squalene, carrageenan, potash soap, medicated soap, alkyldiaminoethylglycine hydrochloride solution, reduced lanolin, sulfated castor oil potassium salt· alkylbenzenesulfonate mixture, diisopropanolamine, triethanolamine, diethyl sebacate, ethanol, glycerol, cetanol, myristyl alcohol, octyl dodecanol, oleyl alcohol, stearyl alcohol, cetostearyl alcohol, stearyl alcohol·polyoxyethylene stearyl ether mixture, cetanol·polyoxyethylenecetyl ether mixed wax, cetanol·polysorbate 60 mixed wax, cetanol·polyoxyethylene sorbitan monostearate mixed wax, cetostearyl alcohol·sodium cetostearyl sulfate mixture, pentaerythrityl citric acid higher fatty acid ester·beeswax·nonionic emulsifier mixture, dicetyl phosphate, sodium N-lauroyl-L-glutamate, benzoin tincture, medium chain fatty acid triglyceride, sodium N-acyl-L-glutamate, benzalkonium chloride, potassium hydroxide, sodium hydroxide, soybean lecithin, purified soybean lecithin, purified lanolin, talc, sodium cetylsulfate, sodium laurylsulfate, hydrogenated soybean phospholipid, partially hydrogenated soybean phospholipid, hydrogenated lanolin alcohol, and pectin.

Examples of the buffer include citric acid, citric anhydride, sodium citrate, tartaric acid, succinic acid, dl-malic acid, fumaric acid, maleic acid, lactic acid, sodium lactate solution, boric acid, borax, acetic acid, glacial acetic acid, sodium acetate, phosphoric acid, dibasic sodium phosphate, potassium dihydrogen phosphate, sodium dihydrogen phosphate, anhydrous sodium hydrogen phosphate, anhydrous sodium dihydrogen phosphate, benzoic acid, sodium benzoate, primary, secondary or tertiary phosphate (e.g., primary sodium phosphate, dibasic sodium phosphate, anhydrous trisodium phosphate, and trisodium phosphate), sodium metaphosphate, ε-aminocaproic acid, sodium hydroxide, sodium carbonate, sodium bicarbonate, ammonium chloride, sodium chloride, benzalkonium chloride, amino acids or salts thereof (e.*g*., glycine and L-arginine), diethanolamine, diisopropanolamine, monoethanolamine, triethanolamine, triethanolamine hydrochloride, sodium triethanolamine phosphate ester solution, chlorobutanol, glucose, and rose oil.

Examples of the transdermal permeability enhancer include triacetin(glyceryl triacetate), crotamiton, urea, ethanol, decylmethylsulfoxide, essential oils, terpene oils (*e.g*., mint oil, orange oil, turpentine oil, L-menthol, d-limonene, menthone, pinene, piperitone, terpinene, terpinolene, terpinol, and carveol), fatty acid esters (*e.g*., glyceryl monolaurate, glyceryl monooleate, cetyl lactate, glycerol monolaurate, glycerol monooleate, propylene glycol monolaurate, propylene glycol monooleate, sorbitan monolaurate, and sorbitan monooleate), dibasic acid diesters (*e.g*., diethylsebacate and diisopropyl adipate), azacycloalkanes (*e.g*., AZONE and 1-(2-(decylthio)ethyl)azacyclopentane-2-one), fatty acids or aliphatic alcohols (*e.g.*, oleic acid, lauric acid, myristic acid, oleyl alcohol, isopropanol, and lauryl alcohol), polyoxyethylene (2) lauryl ether, polyoxyethylene (2) oleyl ether, lauryl diethanolamide, isopropyl myristate, N-hydroxymethyllactate, sorbitol, and squalene.

Examples of the tackifier or tackiness enhancer include natural rubber, crude rubber, RSS No.1 crude rubber, styrene isoprene rubber, styrene-butadiene rubber (SBR), cis-polyisoprene rubber, polyisobutylene rubber, high-cis-polyisoprene rubber, styrene-isoprene-styrene block copolymer (SIS), styrene-isobutylene-styrene block copolymer, styrene-butadiene-styrene block copolymer (SBS), silicone rubber, methyl vinyl ether·maleic anhydride copolymer, acrylic copolymers (*e.g*., (meth)acrylic acid-(meth)acrylate copolymer, acrylic acid-octyl acrylate copolymer, acrylate·vinyl acetate copolymer, 2-ethylhexyl acrylate·vinyl pyrrolidone copolymer solution, 2-ethylhexyl acrylate·2-ethylhexyl methacrylate· dodecyl methacrylate copolymer solution, methyl acrylate·2-ethylhexyl acrylate copolymer resin emulsion, methacrylic acid· n-butyl acrylate copolymer, acrylic acid silk fibroin copolymer resin, and acrylic resin alkanolamine solution), polybutene, maleinated rosin glycerol ester, alicyclic saturated hydrocarbon resins, aliphatic hydrocarbon resins, petroleum resins, terpene resins, higher aqueous polymers (e.g., starch acrylate), hydrophilic polymers (polyacrylic acid, aqueous polyacrylic acid solution, sodium polyacrylate, partially neutralized polyacrylic acid, carboxyvinyl polymer, methylcellulose , carboxymethylcellulose (CMC), sodium carboxymethylcellulose (CMCNa), hydroxyethylcellulose, hydoxypropylcellulose, hydoxypropylmethylcellulose (HPMC) (e.g., hydoxypropylmethylcellulose 2208, hydoxypropylmethylcellulose 2906, and hydoxypropylmethylcellulose 2910), macrogol 400, macrogol 6000, polyvinyl alcohol (PVA), polyvinylpyrrolidone (PVP), sodium alginate, propylene glycol alginate, pectin, zanthan gum, xanthan gum, Locust bean gum, guar gum, arabiano galactan, and sodium hyaluronate), rosin, ultra-pale rosin derivatives, hydrogenated rosin glycerol ester, propylene glycol, dibutylhydroxytoluene, glycerol, glucono-δ-lactone, gum Arabic, gum Arabic powder, ester gum, gum dammer, hydrated lanolin, gelatin, dextrin, casein sodium, collodion, gum tragacanth, tragacanth powder, wheat starch, white vaseline, vaseline· lanolin alcohol mixture, carnauba wax, starch syrup, aluminum magnesium silicate, light anhydrous silicate, corn oil, castor oil, sulfated castor oil potassium salt·alkylbenzenesulfonate mixture, benzyl acetate, talc, and polyisobutylene.

Examples of the adhesive include crosslinked or non-crosslinked acrylic coplymers, vinyl acetate adhesives, polyisobutylene, neoprene, polybutadiene, polyisoprene, ethylene vinyl acetate copolymer, polysiloxane, polyacrylate, polyurethane, polyether block amide copolymer, and styrene rubber block copolymer.

Examples of the skin irritancy mitigator include glycerol and crotamiton.

Examples of additional additives include slurry agents (e.g., gelatin); powdered excipients (*e.g*., kaolin, bentonite, and zinc oxide); petroleum resins (*e.g*., Quinton and Arkon); D-sorbitol; D-sorbitol solution; white sugar; surfactants (*e.g.*, polyoxyethylene hydrogenated castor oil 20, polyoxyethylene hydrogenated castor oil 60, polyoxyethylene sorbitol fatty acid esters (*e.g.*, polysorbate 20, polysorbate 60, polysorbate 80, and polyoxyethylene sorbitan monolaurate), polyoxyethylene fatty acid esters (e.g., polyoxyl stearate 40), sorbitan fatty acid esters (*e.g*., sorbitan monooleate, sorbitan trioleate, sorbitan monolaurate, and sorbitan sesquioleate), self-emulsifying glycerol monostearate, glycerol monostearate, sorbitan monostearate, sucrose fatty acid ester, macrogol 400, lauromacrogol , sodium polyoxyethylene lauryl ether phosphate, polyoxyethylene oleyl ether phosphate, polyoxyethylene nonylphenyl ether, polyoxyethylene octylphenyl ether, polyoxyethylene polyoxypropylene glycols (polyoxyethylene (120) polyoxypropylene (40) glycol, polyoxyethylene (160) polyoxypropylene (30) glycol, and polyoxyethylene (20) polyoxypropylene (20) glycol), polyoxyethylene polyoxypropylene decyl tetradecyl ether, alkyl aryl polyether alcohol, polyoxyethylenecetyl ether, polyoxyethylene oleyl amine, polyoxyethylene sorbitol beeswax, diethanolamide laurate, stearyl alcohol, dibasic acid diesters (e.g., diethyl sebacate), squalane, N-cocoyl-L-arginine ethyl ester DL-pyrrolidone carboxylate, sodium N-cocoyl-N-methylaminoethyl sulfonate, cetanol, cetomacrogol 1000, and sodium laurylsulfate; preservatives and/or antioxidants (e.g., parabens (e.g., methylparaben), sorbic acid and salts thereof, butylhydroxyanisole (BHA), dibutylhydroxytoluene (BHT), nordihydroguaiaretic acid, guaiacol esters, 1,3-butylene glycol, and sodium dehydroacetate), salts that form trivalent metal ions (*e.g*., aluminum chloride, alum, and aluminum allantoinate); humectants (alkaline earth metals (e.g., magnesium chloride), urea, glycerol, and sodium hyaluronate); flavoring agents (mint oil, orange oil, chamomile oil, spearmint oil, clove oil, turpentine oil, pine oil, mint water, deodar oil, bergamot oil, eucalyptus oil, lavender oil, rose water, rose oil, anthemis nobilis flower oil, Peruvian balsam, d-camphor, dl-camphor, d-borneol, dl- borneol, dl-menthol, 1-menthol, geraniol, methyl salicylate, cinnamaldehyde, and piperonal); solvents (*e.g.*; organic solvents (*e.g.*, methanol, ethanol, ethyl acetate, n-butyl acetate, isopropanol, diethyl ether, 2-ethyl-1,3-hexanediol, methanol denatured alcohol, methyl isobutyl ketone, and methyl ethyl ketone), hydrochloric acid, water, physiological saline, aqueous ethanol, unsaturated fatty acids (*e.g.*, oleic acid), synthetic squalane, dipropylene glycol, ethylene glycol salicylate, petroleum benzin, trichloroethane, and 8-acetylsucrose denatured alcohol).

To ensure that the drug is effectively transferred to the skin from a transdermal preparation, in particular, a patch, the patch must be firmly secured to the skin surface. However, an excessively adherent patch can peel the stratum corneum upon removal after use, causing skin irritancy. To reduce the physical skin irritancy while ensuring the adherence of the patch to the skin, oily gel adherence technique may be employed in the present invention. Such an oily gel adherence technique comprises a readily peelable adsorbent, a tackifier to form an adhesive layer for securing the patch to the skin surface, and a liquid component. The readily peelable adsorbent may be a crosslinked acrylic adhesive polymer, ethylene·acetic acid·vinyl copolymer, or ethylene-ethylacrylate copolymer. The tackifier to form an adhesive layer for securing the patch to the skin surface may be natural rubber-based, synthetic rubber-based, acryl-based, silicone-based, or vinyl ether-based. The liquid component may be a fatty acid ester.

The transdermal preparation of the present invention is a patch comprising KRP-197 and an external preparation base. Specifically, the transdermal preparation is provided in the form of a single-adhesive-layer type or a reservoir type.

The transdermal therapeutic preparation of the single adhesive layer type provided in accordance with the present invention (referred to as "single adhesive layer-type transdermal preparation," hereinafter) consists of an adhesive layer and a structural body consisting of a support and a peelable liner for protecting the adhesive layer. The adhesive layer contains the active ingredient KRP-197 and is formed of a base or combination of bases selected from the above-described external preparation bases. One embodiment of the single adhesive layer-type transdermal preparation is shown in cross-section in Fig. 1.

While the support for forming the structural body of the single adhesive layer-type transdermal preparation may be any suitable support, it is preferably flexible enough to not cause significant discomfort when applied to the skin surface. Such a support may be a single-layer or multilayer film formed of materials including plastic films (e.g., polyethylene, polyethylene terephthalate, polyurethane, ethylene vinyl acetate, polypropylene, polyester, poly vinyl acetate, and ethylene-vinylacetate copolymer), metal foils (e.g., aluminum foil), nonwoven fabric, cotton cloth, woven fabric, and paper. While the peelable liner may be any suitable liner, it is preferably a paper strip or plastic film treated with a silicone resin or fluorine resin to make it peelable.

The present invention encompasses not only single adhesive layer-type transdermal preparations in which the adhesive layer comprising the active ingredient KRP-197 and the external preparation base contains KRP-197 in its dissolved form alone, but also single adhesive layer-type transdermal preparations in which the adhesive layer contains both dissolved and non-dissolved forms of KRP-197.

In general, a drug in its non-dissolved form is not readily absorbed through the skin: The more dissolved form is present in the adhesive layer, the more drug is absorbed through the skin at an early stage and the longer the effect can last. In other words, how long the pharmacological effect can last is a function of the saturation solubility of the drug in the external preparation base. An external preparation base with low drug solubility may not provide sufficiently long-lasting effect or effective blood concentration of the drug. To ensure sustained effect of the drug, either an adhesive layer with higher solubility may be used, the thickness of the drug-containing adhesive layer may be increased, the amount of the drug may be increased, or the contact area of the adhesive layer with the skin surface may be increased. Although each of these measures can increase the dose of the delivered drug, each has disadvantages in terms of discomfort, adhesion, skin irritancy, and economy.

The concentration of the dissolved drug in the adhesive layer directly affects the rate of transdermal drug absorption and decreases as the drug is absorbed by the skin. Since the drug present in excess of the saturation solubility of the drug in the adhesive layer used is dispersed through the adhesive layer as non-dissolved form, the amount of the dissolved drug in the adhesive layer depends on the type of the external preparation base used.

On the other hand, the non-dissolved form of the drug serves to supplement the dissolved form as the dissolved form in the adhesive layer is absorbed by the skin and diminishes. This helps to maintain high transdermal absorption rate and, thus, effective blood concentrations of drug over a prolonged period of time.

According to the present invention, adhesive layers with high saturation solubility of the drug can be selected to make preparations containing the dissolved form of the active ingredient KRP-197 alone or preparations containing both the dissolved and non-dissolved forms of KRP-197. In this manner, single adhesive layer-type transdermal preparations can be provided that ensures high transdermal drug absorption, stable kinetics of the drug in the blood, and sustained effect of the drug.

As used herein, the term "dissolved form of KRP-197" means that KPR-197 is present in the adhesive layer in a dissolved state. Specifically, this means that the resulting adhesive layer is uniform with no crystallized KRP-197 observed visually or by light microscopy. It is preferred that the drug remain uncrystallized and completely dissolved in the adhesive layer at higher concentrations. This ensures high transdermal absorption rate of the drug at an early stage of application as well as long-lasting effective blood concentrations of the drug. The dissolved form of KRP-197 is present in the adhesive layer in an amount of about 0.01 wt% or more, preferably about 1 wt% or more, relative to the weight of the adhesive layer.

The tackifier for use in the external preparation base to form the adhesive layer may be any tackifier that can dissolve KRP-197 in an amount of about 0.01 wt% or more of the weight of the adhesive layer. Preferred examples of the tackifier include acryl-based tackifiers, silicone-based tackifiers and synthetic rubber-based tackifiers. Among acryl-based tackifiers are solvent-free type, emulsion type, and solvent type. Acryl-based tackifiers are formed of acrylic polymers, including homopolymers and copolymers of (meth)acrylic acid alkyl esters. To maintain high concentrations of dissolved KRP-197, an additive is preferably used. This additive may be any additive that is compatible with the tackifier, dissolves sufficient amounts of KRP-197, and does not separate from the tackifier over time.

The additive to the adhesive layer containing the dissolved form of KRP-197 must dissolve sufficient amounts of the drug and should not cause adverse effects on the adhesive layer, such as the drug forming ionic bonds with the functional groups of the additive and thereby being trapped in the adhesive layer.

As used herein, the term "the dissolved and non-dissolved forms of KRP-197" refers to the state in which KRP-197 is present in both of dissolved state and non-dissolved state, specifically in both of dissolved state and crystallized state or uncrystallized state. As the dissolved KRP-197 in the adhesive layer is rapidly absorbed by the skin and diminishes, the non-dissolved form of KRP-197 immediately redissolves to supplement the dissolved form. The dissolved KRP-197 is absorbed through the skin and, thus, the effect of the drug is maintained. The ratio of the rate at which the non-dissolved KRP-197 diminishes to the rate at which the total KRP-197 diminishes in the adhesive layer is preferably about 0.1 or higher. If this ratio is too small, the dissolved drug diminishes much faster than the redissolving of the non-dissolved drug, resulting in diminishing effect.

The ratio of the amount of the non-dissolved KRP-197 to the amount of the dissolved KRP-197 present in the adhesive layer is preferably about 0.1 or higher. If this ratio is too small, the effect of the drug does not last long. If this ratio is larger than, for example, 10, considerable amounts of non-dissolved KRP-197 crystallize on the surface of the adhesive layer. As a result, the amount of the dissolved KRP-197 that is exposed to the skin surface is decreased, leading to a decreased rate of transdermal absorption. Furthermore, the adhesion of the adhesive layer to the skin is lost. Thus, additives used in the adhesive layer may differ depending on the physical properties of the drug.

The non-dissolved form of KRP-197 is preferably crystallized form of KRP-197, or crystallized KRP-197.

In the production of the single adhesive layer-type transdermal preparation, KRP-197 is used by dissolving in a solvent. Examples of the solvent are a solvent or a mixture of solvents selected from water, lower alcohols (e.g., ethanol and isopropyl alcohol), C2 to C5 alkane diols (e.g., glycerol), C2 to C5 alkane triols (e.g., propylene glycol), chloroform, and volatile solvents that can dissolve KRP-197. In making the preparations in which the non-dissolved form and the dissolved form of KRP-197 are present together, the type and size of the resulting crystals may vary depending on the type of the solvent and the time and temperature at which the preparations are dried.

In making the adhesive layer, KRP-197 and the tackifier are dissolved in the solvent, and the solution is dried for example at about 10 to 50°C. This gives an adhesive layer in which fine crystals with the average particle size of 1 to 50µm are uniformly dispersed.

The reservoir-type transdermal preparation of the present invention according to claim 3 comprises a mixture of the active ingredient KRP-197 and a base or combination of bases selected from the above-described external preparation bases (reservoir content); and a structural body comprising a membrane for controlling drug permeation, an adhesive layer, a support that can hold the reservoir content, and a peelable liner. One embodiment of the reservoir-type therapeutic transdermal preparation is shown in cross-section in Fig. 2.

While the support for forming the structural body of the reservoir-type may be any suitable support that can hold the reservoir content, supports similar to those used in the single adhesive layer-type transdermal preparations may be used. While the peelable liner may be any suitable liner, liners similar to those used in the single adhesive layer-type transdermal preparations may be used. The drug permeability control membrane may be a fine porous membrane, such as fine porous polypropylene, or other membrane materials that can control the drug permeability. The adhesive layer may be formed of a rubber-based, acryl-based or silicone-based tackifier that is permeable to the drug. It may be a double-sided tape. The adhesive layer may also serve as the drug permeability control membrane.

In the production of the reservoir-type transdermal preparation, KRP-197 is used by dissolving in a solvent. Examples of the solvent are a solvent or a mixture of solvents selected from water, lower alcohols (*e.g*., ethanol and isopropyl alcohol), C2 to C5 alkane diols (*e.g*., glycerol), C2 to C5 alkane triols (*e.g*., propylene glycol) and chloroform.

When applied to the skin, the preparation of the present invention may be changed for a new one once a day or once in every two to seven days. The preparation may be applied only one day during a two to seven day period, or it may be applied at bedtime or when necessary. The preparation can be applied, for example, behind the ears or to the surface of the arms, abdomen, chest, back, or legs.

The dose of KRP-197 delivered by a single application of the preparation of the present invention is preferably from about 0.01 mg to about 50 mg, and more preferably from about 0.1 mg to about 10 mg.

KRP-197 for use in the present invention acts as a selective antagonist for muscarinic M3 and M1 receptors found in smooth muscles of bladder, trachea, and digestive tract, and can thus be used in the prevention or treatment not only of increased urinary frequency and urinary incontinence, but also of asthma, chronic obstructive Pulmonary Disease (COPD), irritable bowel syndrome (IBS), and various other diseases. Thus, the transdermal preparation of the present invention containing KRP-197 as an active ingredient has the following advantages: The side effects caused by temporarily high blood concentrations of drug occurring in administration of oral preparations and injections can be avoided; sustained drug absorption through the skin requires fewer times of administration; and application can be immediately stopped in the event of side effects. Providing sustained effect, the transdermal preparation of the present invention is highly useful in the prevention and/or treatment of increased urinary frequency and urinary incontinence, asthma, COPD, IBS, and various other diseases and can be used to improve the quality of lives of elderly people or patients to whom oral administration can be hardly applied.

While KRP-197 has been described as one example of the active ingredients for use in the transdermal preparation of the present invention, any other muscarinic antagonist may be used as the active ingredient. Examples of other muscarinic antagonists other than KRP-197 for use in the present invention include tolterodine ((+)-(R)-2-[α-[2-(diisopropylamino)ethyl]benzoyl]-p-cresol tartrate), darifenacin ((S)-1-[2-(2,3-dihydro-5-benzofuranyl)ethyl]-α,α-diphenyl-3-pyrrolidine acetoamide hydrobromide), solifenacin ((+)-(1S,3'R)-quinuclidin-3'-yl-phenyl-1,2,3,4-tetrahydroisoquinoline-2-carboxylate succinate), oxybutynin hydrochloride (4-diethylamino-2-butynyl(±)-α-cyclohexyl-α-phenylglycolate hydrochloride), propiverine hydrochloride (1-methyl-4-piperidyl-α,α-diphenyl-α-n-propoxyacetate hydrochloride), terodiline hydrochloride ((±)-N-t-butyl-1-methyl-3,3-diphenylpropylamine hydrochloride), and flavoxate hydrochloride (2-piperidinoethyl-3-methyl-4-oxo-2-phenyl-4H-1-benzopyrane-8-carboxylate hydrochloride).

The transdermal preparation of the present invention containing KRP-197 as an active ingredient may be provided, other than in the form of the single adhesive layer-type transdermal preparation and reservoir-type transdermal preparation, in the form of ointment, gel, cream, emulsion, lotion or liquid preparation. Plaster and tape preparations are particularly preferred. These transdermal preparations can be prepared by selecting a base or combination of bases from the above-described external preparation bases and/or other external preparation bases suitable for use in each preparation and by using any technique commonly used in the production of pharmaceutical preparations or cosmetics.

Examples of other external preparation bases suitable for use in gel preparations include suspendable bases, such as diisopropyl adipate, diethyl sebacate, isopropyl myristate, propylene glycol, 1,3-butylene glycol, polyethylene glycol, medium chain fatty acid triglycerides, propylene glycol monocaprate, crotamiton, diethylamine, triethanolamine, diethanolamine, N-methylpyrrolidone, and L-menthol.

The present invention will now be described in detail with reference to examples, which are not intended to limit the scope of the invention in any way.

Examples of single adhesive layer-type transdermal preparation are presented in Examples 1 through 4, and examples of reservoir-type transdermal preparation are presented in Examples 5 and 6.

### [Example 1]

0.1 parts by weight of KRP-197 were dissolved in 37.5 parts by weight of chloroform. To this solution, 3.95 parts by weight of styrene-isobutylene-styrene block copolymer (Trade name Kraton D1107CU, Kraton Polymers Japan Co., Ltd.), 4.95 parts by weight of a ultra-pale rosin ester (Trade name Ultra pale rosin ester KE-311, Arakawa Chemical Co., Ltd.) and 1 part by weight of isopropyl myristate were added, and the mixture was stirred to dissolve the components. This gave an adhesive layer. Using a Baker applicator with a thickness setting of 10 scales, the resulting adhesive layer was spread over a support (Trade name 3M Scotchpak 9742 Release Liner, Sumitomo 3M Co., Ltd.) on its polyester surface. Three strips of double-sided tape were stuck to each leg of the applicator. The coated support was dried at room temperature for about 15min and was further dried in a drier at 60°C for about 15min. The adhesive surface was then laminated with a peelable liner (Trade name 3M Scotchpak 9742 Release Liner, Sumitomo 3M Co., Ltd.) with its fluorine polymer surface facing the adhesive layer. This gave a 0.1mm thick, single adhesive layer-type transdermal preparation containing 0.1 mg/cm² active ingredient.

### [Example 2]

0.1 parts by weight of KRP-197 were dissolved in 37.5 parts by weight of chloroform. To this solution, 2.85 parts by weight of styrene-isobutylene-styrene block copolymer (Trade name Kraton D1107CU, Kraton Polymers Japan Co., Ltd.), 5.05 parts by weight of a ultra-pale rosin ester (Trade name Ultra pale rosin ester KE-311, Arakawa Chemical Co., Ltd.), 1 part by weight of isopropyl myristate and 1 part by weight of liquid paraffin were added, and the mixture was stirred to dissolve the components. This gave an adhesive layer. Using a Baker applicator with a thickness setting of 10 scales, the resulting adhesive layer was spread over a support (Trade name 3M Scotchpak 9742 Release Liner, Sumitomo 3M Co., Ltd.) on its polyester surface. Three strips of double-sided tape were stuck to each leg of the applicator. The coated support was dried at room temperature for about 15min and was further dried in a drier at 60°C for about 15min. The adhesive surface was then laminated with a peelable liner (Trade name 3M Scotchpak 9742 Release Liner, Sumitomo 3M Co., Ltd.) with its fluorine polymer surface facing the adhesive layer. This gave a 0.1mm thick, single adhesive layer-type transdermal preparation containing 0.1 mg/cm² active ingredient.

### [Example 3]

0.1 parts by weight of KRP-197 were dissolved in 37.5 parts by weight of chloroform. To this solution, 3.95 parts by weight of styrene-isobutylene-styrene block copolymer (Trade name Kraton D1107CU, Kraton Polymers Japan Co., Ltd.), 4.95 parts by weight of a ultra-pale rosin ester (Trade name Ultra pale rosin ester KE-311, Arakawa Chemical Co., Ltd.) and 1 part by weight of oleyl alcohol were added, and the mixture was stirred to dissolve the components. This gave an adhesive layer. Using a Baker applicator with a thickness setting of 10 scales, the resulting adhesive layer was spread over a support (Trade name 3M Scotchpak 9742 Release Liner, Sumitomo 3M Co., Ltd.) on its polyester surface. Three strips of double-sided tape were stuck to each leg of the applicator. The coated support was dried at room temperature for about 15min and was further dried in a drier at 60°C for about 15min. The adhesive surface was then laminated with a peelable liner (Trade name 3M Scotchpak 9742 Release Liner, Sumitomo 3M Co., Ltd.) with its fluorine polymer surface facing the adhesive layer. This gave a 0.1mm thick, single adhesive layer-type transdermal preparation containing 0.1 mg/cm² active ingredient.

### [Example 4]

0.1 parts by weight of KRP-197 were dissolved in 37.5 parts by weight of chloroform. To this solution, 3.95 parts by weight of styrene-isobutylene-styrene block copolymer (Trade name Kraton D1107CU, Kraton Polymers Japan Co., Ltd.), 4.9 parts by weight of a ultra-pale rosin ester (Trade name Ultra pale rosin ester KE-311, Arakawa Chemical Co., Ltd.), 1 part by weight of olive oil and 0.5 parts by weight of L-menthol were added, and the mixture was stirred to dissolve the components. This gave an adhesive layer. Using a Baker applicator with a thickness setting of 10 scales, the resulting adhesive layer was spread over a support (Trade name 3M Scotchpak 9742 Release Liner, Sumitomo 3M Co., Ltd.) on its polyester surface. Three strips of double-sided tape were stuck to each leg of the applicator. The coated support was dried at room temperature for about 15min and was further dried in a drier at 60°C for about 15min. The adhesive surface was then laminated with a peelable liner (Trade name 3M Scotchpak 9742 Release Liner, Sumitomo 3M Co., Ltd.) with its fluorine polymer surface facing the adhesive layer. This gave a 0.1mm thick, single adhesive layer-type transdermal preparation containing 0.1 mg/cm² active ingredient.

### [Example 5]

0.2 parts by weight of KRP-197 were dispersed in 0.05 parts by weight of L-menthol in 9.75 parts by weight of an ethanol(99.5)/water mixture (mass ratio = 1 : 1.3) to give a KRP-197 dispersion. A double-sided tape (Trade name Double-sided paper adhesion tape, Kokuyo Co., Ltd.) was used as the drug permeability control membrane and the adhesive layer.

### [Example 6]

To 0.2 parts by weight of KRP-197, 0.05 parts by weight of L-menthol, 0.2 parts by weight of HPMC, and 9.75 parts by weight of an ethanol(99.5)/water mixture (mass ratio = 1 : 1.3) were added and the components were dispersed to give a material to serve as the reservoir content. 0.9 g of this material were stuffed in a support (Trade name Cosmopack PTP, Kanae Co., Ltd.) and the opening of the support was sealed with a strip of double-sided tape (Trade name Double-sided paper adhesion tape, Kokuyo Co., Ltd.) to serve both as the drug permeability control membrane and the adhesive layer. This gave a reservoir-type transdermal preparation. [Comparative Example 1]

To 0.2 parts by weight of KRP-197, 0.1 parts by weight of gentamicin sulfate and 9.7 parts by weight of water were added and the components were dispersed to obtain a sample solution for evaluating the skin permeability of KRP-197 alone.

### <Test Example 1> (in vitro skin permeability test)

Excisions of shaved abdominal skin of male hairless rats (body weight = 229.7 to 328.1g) were each mounted on a horizontal diffusion cell (effective diffusion area = 0.95cm², cell volume = 2.5mL). As depicted in Fig. 3(a), each of the single adhesive layer-type transdermal preparations of Examples 1 through 4 was applied to the stratum corneum of the shaved abdominal skin. The applied area was approximately 0.95cm². In Example 5, the KRP-197 suspension was placed in the cell on the stratum corneum side, and the double-sided tape to serve as the drug permeability control membrane and the adhesive layer was applied to the stratum corneum of the shaved abdominal skin, as depicted in Fig. 3(b). In Comparative Example 1, the sample solution for evaluating the skin permeability of KRP-197 alone was placed in the cell on the stratum corneum side, as depicted in Fig. 3(c). In each of Examples 1 through 5 and Comparative Example 1, phosphate buffer solution (pH 7.4) containing 1% gentamicin sulfate was placed in the cell on the dermis side. The phosphate buffer solution (pH 7.4) containing 1% gentamicin sulfate placed in the cell on the dermis side was sampled at intervals and each sample was analyzed by HPLC for the amount of KRP-197. The results of the analysis were used to plot the cumulative amounts of KRP-197 that had permeated through the skin. The rate of skin permeation was then determined from the slope of the plot. The results are shown in Table 1.

**Table 1**

| **Skin permeation rate (µg/hour/cm**^{**2**}**)** | |
|---|---|
| Example 1 | 2.76 |
| Example 2 | 1.48 |
| Example 3 | 1.90 |
| Example 4 | 1.30 |
| Example 5 | 6.91 |
| Comparative Example 1 | 0.58 |

As can be seen from the results of Table 1, the low skin permeability of KRP-197 (Comparative Example 1) was improved in each of Examples. Of the single adhesive layer-type transdermal preparations tested, the preparation of Example 1, which used styrene-isobutylene-styrene block copolymer, ultra-pale rosin ester, and isopropyl myristate in the base of the adhesive layer, exhibited the highest skin permeability of KRP-197. The rate of skin permeation of Example 1 was about five times higher than that of Comparative Example 1. The reservoir-type transdermal preparation of Example 5, in which L-menthol, ethanol (99.5) and water were used to form the base of the reservoir content and a double-sided tape was used to serve both as the adhesive layer and the drug permeability control membrane, showed a skin permeation rate about 12 times higher than that of Comparative Example 1.

### <Test Example 2> (in vivo skin application test)

The abdominal skin of male hairless rats (body weight = 202.9 to 252.3g) was shaved by an electric razor. A piece of the single adhesive layer-type transdermal preparation of Example 1 or the reservoir-type transdermal preparation of Example 6, prepared based on the results of in vitro skin permeability test (Example 5), was applied to each rat in the shaved abdominal skin (Example 1 was applied over an about 3cm² area, Example 6 over an about 9cm² area). The preparations were secured by a strip of commercial surgical tape to prevent their peeling during the test. Blood samples were collected from subclavian vein at intervals and serum was separated. The serum concentrations of KRP-197 were determined by LC-MS/MS analysis. The results are shown in Fig. 4.

As can be seen from Fig. 4, the single adhesive layer-type transdermal preparation of Example 1 and the reservoir-type transdermal preparation of Example 6 each showed sustained release of KRP-197.

### <Test Example 3> (Evaluation of primary skin irritation by Draize method)

Male rabbits (body weight = 2.90 to 2.96kg) were used. On the day before (24 hours before) the application of the preparations of Examples 1 and 6, the dorsal skin of each application sites were directly used as "normal skin" and the other two were scratched in the stratum corneum with a syringe needle (to a depth that does not cause bleeding) and used as "damaged skin." The preparation of Example 1 was applied to one of the normal skin sites and one of the damaged skin sites and the preparation of Example 6 was applied to the other of the normal skin sites and the other of the damaged skin sites (Example 1 was applied over an about 3cm² area, Example 6 over an about 9cm² area). The preparations were each secured by a strip of adhesive bandage (Nichiban). The preparations were removed after 48 hours and the skin conditions were evaluated immediately after the removal and after 24 and 48 hours according to the Draize's criteria shown in Table 2.

**Table 2**

| A. Erythema and eschar formation | | |
|---|---|---|
| | No erythema | 0 |
| | Very slight erythema (barely perceptible) | 1 |
| | Well-defined erythema | 2 |
| | Moderate to severe erythema | 3 |
| | Severe erythema (beet redness) and slight eschar formation | 4 |

| B. Edema formation | | |
|---|---|---|
| | No edema | 0 |
| | Very slight edema (barelyperceptible) | 1 |
| | Slight edema (edges of area well defined by raising) | 2 |
| | Moderate edema (raised approx. 1 mm) | 3 |
| | Severe edema (raised more than 1mm and extending beyond area of exposure) | 4 |

The scores of the normal skin sites and the damaged skin sites were individually added and the totals were divided by 6 to obtain primary skin irritation indices. The safety classification was as follows: a preparation with a primary skin irritation index of 0 to 2 was rated as a weak irritant; a preparation with a primary skin irritation index of 3 to 5 was rated as a moderate irritant; and a preparation with a primary skin irritation index of 6 or higher was rated as a strong irritant. The results are shown in Table 3.

**Table 3**

| Test Examples | Classification | Scores | | | Primary skin irritation index | Safety class |
|---|---|---|---|---|---|---|
| | | 0 hr | 24 hrs | 48 hrs | | |
| Example 1 | Normal skin | 1.33 | 1 | 0.67 | 0.89 | Mild |
| | Damaged skin | 1 | 1 | 0.33 | | |
| Example 6 | Normal skin | 1 | 1 | 1 | 1.06 | Mild |
| | Damaged skin | 1.33 | 1 | 1 | | |

As can be seen from Table 3, the single adhesive layer-type transdermal preparation of Example 1 and the reservoir-type transdermal preparation of Example 6 were each rated as a weak irritant and thus posed no serious irritation problems.

### INDUSTRIAL APPLICABILITY

According to the present invention, there is provided a transdermal preparation that causes less skin irritancy and ensures efficient and sustained skin absorption of KRP-197, which otherwise is hardly absorbed through the skin. The preparation of the present invention circumvents the problem of solid oral preparations (*e.g*., tablets): the solid oral preparations are not suitable for administering drugs to children and aged people who have a low ability to maintain drug effect and swallow. The preparation of the present invention also circumvents the problems associated with commonly used non-oral preparations (*e.g*., injections and external preparations), such as follows: non-oral preparations may accompany pain; drugs cannot be eliminated once administered by non-oral preparations; non-oral preparations are difficult to use at home; and non-oral preparations, generally applied by patients themselves or their caretakers , result in poorly controlled drug delivery or dosage, leading to a reduced chance of proper drug use. These advantages should make the preparation of the present invention widely accepted by patients of varying ages, their caretakers, and medical personnels.

## Claims

1. A transdermal preparation containing a muscarinic receptor antagonist and an external preparation base.

2. The preparation according to claim 1, wherein the muscarinic receptor antagonist is one or two or more selected from 4-(2-methyl-1-imidazolyl)-2,2-diphenylbutylamide, (+)-(R)-2-[α-[2-(diisopropylamino)ethyl]benzoyl]-p-cresol tartrate, (S)-1-[2-(2,3-dihydro-5-benzofuranyl)ethyl]-α,α-diphenyl-3-pyrrolidine acetamide hydrobromide, (+)-(1S,3'R)-quinuclidin-3'-yl-phenyl-1,2,3,4-tetrahydroisoquinoline-2-carboxylate succinate, 4-diethylamino-2-butynyl(±)-α-cyclohexyl-α-phenylglycolate hydrochloride, 1-methyl-4-piperidyl-α,α-diphenyl-α-n-propoxyacetate hydrochloride, (±)-N-t-butyl-1-methyl-3,3-diphenylpropylamine hydrochloride, and 2-piperidinoethyl-3-methyl-4-oxo-2-phenyl-4H-1-benzopyran-8-carboxylate.

3. The preparation according to claim 2, wherein the muscarinic receptor antagonist is 4-(2-methyl-1-imidazolyl)-2,2-diphenylbutylamide.

4. The preparation according to claim 1 for use as a prophylactic or therapeutic agent for preventing or treating a disorder in patients with overactive bladder, the disorder selected from increased urinary frequency, urinary incontinence, asthma, chronic airway obstruction, and irritable bowel syndrome.

5. The preparation according to claim 4, wherein the disorder is increased urinary frequency and urinary incontinence in patients with overactive bladder.

6. The preparation according to claim 1, provided in the form of patch.

7. The preparation according to claim 6, provided in the form of a single adhesive layer-type or a reservoir-type transdermal preparation.

8. The preparation according to claim 7, containing as an active ingredient a dissolved form of 4-(2-methyl-1-imidazolyl)-2,2-diphenylbutylamide alone.

9. The preparation according to claim 7, containing as active ingredients dissolved and non-dissolved forms of 4-(2-methyl-1-imidazolyl)-2,2-diphenylbutylamide.

10. The preparation according to claim 1, wherein the external preparation base is a base or combination of bases selected from the group consisting of an amphipathic solubilizing agent, a suspension base, a softener, an emulsifier, a buffer, a transdermal permeability enhancer, a tackifier, a tackiness enhancer, an adhesive, a skin irritancy mitigator, and an additive.

11. The transdermal preparation for treating increased urinary frequency and urinary incontinence according to claim 1, containing as an active ingredient 4-(2-methyl-1-imidazolyl)-2,2-diphenylbutylamide and comprising an external preparation base and a structural body.

12. The transdermal preparation for treating increased urinary frequency and urinary incontinence according to claim 11, which is of a single adhesive layer type, comprising:
an adhesive layer formed of 4-(2-methyl-1-imidazolyl)-2,2-diphenylbutylamide in conjunction with a single or combination of the external preparation bases; and
a structural body comprising a support and a peelable liner.

13. The transdermal preparation for treating increased urinary frequency and urinary incontinence according to claim 11, which is of a reservoir type, comprising:
a mixture of 4-(2-methyl-1-imidazolyl)-2,2-diphenylbutylamide and a single or combination of the external preparation bases; and
a structural body comprising a membrane for controlling drug permeation, an adhesive layer, a support, and a peelable liner.

14. The transdermal preparation for treating increased urinary frequency and urinary incontinence according to any one of claims 11 to 13, wherein the external preparation base comprises a compound or combination of compounds selected from the group consisting of a water-soluble polymer, a fat-soluble polymer, a fatty acid, a fatty acid ester, a fatty acid metal salt, animal or plant fats and oils, an alcohol, a terpene compound, and water.
